# EUROPEAN PATENT SPECIFICATION

(11) **EP 0 350 147 B1**
(45) Date of publication and mention of the grant of the patent: **12.07.1995**
(21) Application number: 89303900.8
(22) Date of filing: 19.04.1989
(51) Int. Cl.: A61L 31/00, A61B 19/04

(54) **Surgical gloves**
Chirugische Handschuhe
Gants chirurgicaux

(30) Priority: 23.04.1988 GB 8809660; 23.04.1988 GB 8809661; 25.04.1988 GB 8809709
(43) Date of publication of application: 10.01.1990
(73) Proprietor: SMITH & NEPHEW INC., Largo, Florida 34643 (US)
(72) Inventor: Milner, Richard, Bishops Stortford Hertfordshire (GB)
(74) Representative: Gilholm, Stephen Philip

(56) References cited:
- EP-A- 0 141 628
- EP-A- 0 147 970
- EP-A- 0 229 862
- WO-A-86/05391
- FR-A- 2 215 506
- GB-A- 1 034 112
- GB-A- 1 421 100
- GB-A- 1 541 155

## Description

This invention relates to gloves and more particularly to the type of gloves worn by medical practitioners such as surgeons, nurses and other medical or paramedical personnel, to the manufacture of such gloves and to their use.

Conventionally surgical gloves are manufactured from extremely thin elastomeric materials such as natural or synthetic rubbers. These gloves fit closely and tightly over the users hand. One disadvantage which is experienced with this type of glove is that they are sometimes punctured or ruptured in use. The presence of any small hole such as that caused by a surgical needle or other surgical instrument can result in contamination and infection at the operation site by transfer of bacteria from the inside of the glove to the open wound or to the surgical instruments. Also if body fluids of the patient carry viable bacteria or viruses these may penetrate through a discontinuity in the glove and if they contact broken skin can cause infection of the surgical personnel involved. It has even been suggested that channels can exist in latex gloves which allow viruses to pass through. Although it is the custom for the medical personnel to scrub their hands vigorously with an antibacterial skin cleanser before donning gloves, the anti-infective agent effect may be short lived and infective agents such as bacteria may regrow beneath the gloves in the moist warm environment. If a glove is punctured in use it may not be recognised and the operation is continued allowing a risk of infection.

It has been suggested that a way of protecting the user of a glove is to provide a coating containing an anti-infective agent (see European Patent Publication No.300814). A secure method of protection is required which does not rely upon maintaining the integrity of a coating (during manufacture and use). Such a method has now been discovered.

It has now been found that by using a glove into which a non-ionic sparingly water soluble antimicrobial agent (for example 2,4,4¹-trichloro-2¹-hydroxydiphenyl ether [triclosan]) has been incorporated, the risk of infection to the patient and the glove wearer is reduced. The level of the antimicrobial agent available on the skin of the wearer is sufficient to inhibit many common bacteria and also to help inhibit certain viruses. It is also believed that such a level would be sufficient to provide an improved barrier to infective agents including certain viruses such as the H.I.V. and Hepatitis B.

Thus in accordance with the invention, there is provided a method for the manufacture of antimicrobial releasing thin polymer gloves, including the step of incorporating an effective amount of a non-ionic, sparingly water soluble antimicrobial agent into the glove material prior to forming the glove.

The present invention also provides an antimicrobial releasing thin polymer glove containing an antimicrobially effective non-ionic, sparingly water soluble antimicrobial agent within the glove material.

The antimicrobial agent is non-ionic at neutral pH values and only sparingly soluble in water. By sparingly water soluble it is meant that the antimicrobial agent has a solubility in water at 20°C of less than 0.1gm/litre, preferably less than 0.05gm/litre.

The gloves may be used as surgeons gloves, as examination gloves or for any other purpose which it is desired to reduce the risk of infection. Aptly the glove is a surgeons glove. Aptly the glove is an examination glove.

The glove material will contain (ie within its material as opposed to residing only on its surface) an antimicrobially effective amount of antimicrobial agent. Suitably the glove material may contain from 0.1 to 10% w/w of antimicrobial agent, more suitably 1 to 5% w/w and preferably about 1.0%w/w.

The material which forms the gloves can be any one of those which are conventionally used for forming gloves especially medical gloves and include natural rubber, polyvinyl chloride and polyurethane. The use of a natural rubber latex to form the glove material is preferred. The use of a non-ionic sparingly soluble antimicrobial agent in a natural rubber latex glove offers a method of overcoming many of the potential problems which could occur with natural rubber latex gloves if they are susceptible to penetration by viruses such as those responsible for AIDS and Hepatitis B.

From the foregoing it will be appreciated that in a preferred aspect this invention provides an antimicrobial releasing thin rubber latex glove containing an antimicrobial non-ionic, sparingly water soluble amount of antimicrobial agent.

The preferred antimicrobial agent is 2,4,4'-trichloro-2'hydroxydiphenyl ether (triclosan).

Thus in a preferred aspect the present invention provides an antimicrobial releasing thin natural rubber latex glove which contains an antimicrobially effective amount of 2,4,4'-trichloro-2'-hydroxydiphenyl ether within the glove material.

These antimicrobial agents are particularly suitable for incorporation into gloves formed from natural rubber latex. It has been observed that natural rubber latices may be coagulated by ionic antimicrobial agent. If articles formed from natural rubber latices are to be treated with such antimicrobial agents, it is after the rubber has been vulcanised that this can be achieved. The nature of the surface of cured rubber articles is such as to be not readily coated or impregnated with for example an antimicrobial agent. Methods of overcoming this problem have included coating the glove with the antimicrobial agent, in a binding agent, modifying the rubber surface to make it responsive to binding the antimicrobial agent or by treating the surface with a solvent to cause the rubber to swell and then impregnating with the antimicrobial agent in the same or another solvent and finally removing the solvent. These processes are difficult to carry out and do not give a consistent product. Surprisingly it has been found that non-ionic, sparingly water soluble antimicrobial agents do not coagulate natural rubber latex. This enables the antimicrobial agent to be uniformly mixed with the rubber latex in the fluid pre-cured state which makes manufacture of rubber articles made therefrom easier as it requires no post-cure operations on the article and can also provide a more consistent product. The low solubility of the antimicrobial agent means that it is not removed during the leaching step in the glove manufacturing process yet the antimicrobial agent is found to be effectively released from both the inside and outside of the glove under conditions of which simulate wear.

Suitable non-ionic, sparingly soluble antimicrobial agents include phenol derivatives such as chlorophene, dichloroxylenol, hexachloraphane; diphenyl derivatives, halogenated hydroxy diphenyl derivatives such as diphenyl ethers for example 2,4,4'-trichloro-2'-hydroxy diphenyl ether (triclosan); and agents such as diacetylamino-azotoluene and triclocarban. The preferred antimicrobial agent is triclosan.

The gloves containing antimicrobial agent may be prepared by mixing the required amount of agent with the glove material, for example an aqueous dispersion such as a natural rubber latex. The agent may have been previously formed into a dispersion by mixing with a little of the latex or by mixing with water and dispersing agents. The rest of the latex may be gradually mixed with this dispersion until a homogenous mixture results. The glove is then formed in the usual manner.

Thus, a former may be first dipped into a coagulant solution and allowed to dry. Thereafter the coated glove former is dipped into the latex, removed, dried and immersed in a leaching bath. After drying the glove may then be 'cured'.

In the process described above the antimicrobial agent is distributed through the whole of the glove material so that the agent may be released from both the inside and the outside of the glove. This is generally much preferred, however, it may be desirable for some purposes to have the agent available only on the inside or wearer-contacting surface of the glove. The agent may be present therefore in the material which forms the last coat on the former, that is becomes the inside of the glove when the glove is removed from the former (this process still ensures that the anti-infective agent is contained within the glove).

In accordance with a further aspect of the invention there is provided a method for the manufacture of antimicrobial releasing thin polymer gloves which includes the step of dipping a glove former into a first polymer solution or latex containing from 0.1 to 10% by weight of a non-ionic sparingly water soluble antimicrobial agent.
In an alternative embodiment, the glove former may may be precoated with a polymeric material such as a polyurethane, and thereafter further coated with the antimicrobial-containing material. In use the coating immediately adjacent the former will become the exterior coating of the glove.

It will be understood that in a preferred aspect this invention provides an antimicrobial releasing thin polymer glove which contains 2,4,4¹ - trichloro - 2¹ - hydroxydiphenyl ether (triclosan).

Such gloves incorporating a non-ionic sparingly water soluble antimicrobial agent offer the user a high degree of protection from common infecting organisms that might penetrate through any discontinuity in the glove. The use of such gloves can reduce the need for extensive pre-sterilization of the skin as the antibacterial agent is effective in reducing skin flora.

The present invention provides a method of reducing the risk of infection which comprises using thin polymer gloves which contain 2,4,4¹ - trichloro - 2¹ - hydroxydiphenyl ether (triclosan). The glove contains an antimicrobially effective amount of triclosan. The triclosan provides protection for 6 to 8 hours.

In another aspect the invention provides a glove which contains a non-ionic, sparingly water soluble antimicrobial agent and which has at least on the inside surface thereof a powder containing antimicrobial agent.

In a preferred aspect the invention provides a glove which contains non-ionic, sparingly water soluble antimicrobial agent and which has at least on the inside surface thereof a powder containing chlorhexidine digluconate.

It is very surprising that chlorhexidine digluconate should be a component of an effective bactericidal dusting powder since it is a hygroscopic or even deliquescent solid and is usually only available as a solution. If used as a solid it would be expected to cause a powder containing it to cake through absorption of water thereby making it ineffective as a dusting powder. However, very surprisingly this is not found to be the case in the dusting powder used in the invention.

In a preferred aspect the present invention provides a glove which contains 2,4,4¹ - trichloro - 2¹ - hydroxydiphenyl ether and which has at least on the inside surface thereof a powder containing chlorhexidine digluconate.

Aptly the gloves may be used as surgeons gloves or as examination gloves.

The powder which contains the chlorhexidine digluconate is preferably one of those which is conventionally used as a lubricating or dusting powder for gloves such as examination gloves or surgeons' gloves. Suitable powders include starches especially maize starch and inorganic powders such as calcium carbonate. The powder comprising starch and chlorhexidine digluconate is particularly effective.

The powder will contain an antimicrobially effective amount of chlorhexidine digluconate. The powder may suitably contain from 0.05 to 10% w/w of chlorhexidine digluconate, more suitably may contain from 0.1 to 8% w/w and preferably contains from 0.15 to 6% w/w.

The powder containing an antimicrobially effective amount may be obtained by methods which comprise mixing of the ingredients. Suitable methods include (a) spraying a solution of the appropriate strength of chlorhexidine digluconate onto a fluidised bed of the powder, and (b) mixing a solution of chlorhexidine digluconate with the powder, drying, grinding and sieving the resulting powder to remove large particles or (c) by freeze drying chlorhexidine digluconate and dry mixing with the powder.

The powder containing chlorhexidine digluconate may be coated onto the inside of the glove in the way lubricating powders are conventionally applied. Suitably each glove may have applied to it from 0.2 to 3 gm of the dusting powder and preferably from 0.4 to 1 gm. The glove is then everted and packaged in a conventional manner.

In a second preferred aspect the present invention provides a glove which contains a non-ionic, sparingly water soluble antimicrobial agent and which has at least on the inside surface thereof a powder containing triclosan.

The powder may contain an antimicrobially effective amount of triclosan. The powder may suitably contain from 0.1 to 10% w/w of triclosan, more suitably may contain from 0.5 to 8% w/w and preferably contains about 1% w/w.

The powder containing an antimicrobially effective amount of triclosan may be obtained by methods which comprise mixing of the ingredients. Suitable methods include (a) mixing a solution of triclosan in acetone with the powder, drying, grinding and sieving the resulting powder to remove large particles and (b) mixing the dry powders together.

The powder containing triclosan may be coated onto the inside of the glove incorporating the non-ionic, sparingly water soluble antimicrobial agent such as triclosan itself in the way lubricating powders are conventionally applied. Suitably each glove may have applied to it from 0.1 to 3 g of the dusting powder and preferably from 0.4 to 1g. The glove is then everted and packaged in a conventional manner.

A further aspect of the invention provides an antimicrobial releasing thin polymer glove containing an antimicrobially effective amount of 2,4,4¹ - trichloro - 2¹ - hydroxy diphenyl ether and on at least the inside surface thereof a powder containing chlorhexidine digluconate-cyclodextrin.

The powder may contain an amount of chlorhexidine digluconate-cyclodextrin complex which contains an equivalent amount of chlorhexidine digluconate as described herein before. The preparation of chlorhexidine digluconate-cyclodextrin complex is described hereinafter. In addition to the complex the powder may contain other materials such as starch.

This powder may be applied to a conventional glove in a conventional manner at a level which is similar to that which the chlorhexidine digluconate powder was applied. This powder may also be beneficially applied to gloves incorporating triclosan as hereinbefore described.

A powder may contain an amount of triclosan-cyclodextrin complex which contains an equivalent amount of triclosan when present in a powder. The preparation of triclosan-cyclodextrin complex is described hereinafter.

Aptly the glove forming material is a natural rubber latex.

Suitable non-ionic, sparingly water soluble antimicrobial agent includes those described herein before.

### Example 1

### Preparation of a Glove

Triclosan was incorporated into a natural rubber latex by mixing the triclosan (42g) with a small quantity of latex to form a paste. The latex paste was gradually diluted with more latex until the required concentration was achieved. The final latex formulation was :

| | |
|---|---|
| Latex (42% solids) | 4858g |
| Triclosan | 42g |

A coagulant solution was prepared consisting of:

| | |
|---|---|
| Calcium nitrate | 12.02% |
| Zinc nitrate | 5.29% |
| Talc | 3.85% |
| Lactic acid | 3.85% |
| Ethanol | 67.4% |
| Methanol | 7.5% |

A glove former, preheated to 112°C was dipped into this solution, removed and air-dried for about 2 minutes. The coated former was then immersed in the pre-vulcanised natural rubber latex containing triclosan. The latex coated former was then withdrawn, air-dried for about 2 minutes and immersed in a leach tank of water at about 70°C for 2 minutes to extract any water soluble materials. The glove was cured, dusted with talc and stripped from the former.

A sample of the glove material was taken and tested for release of triclosan by preparing an assay plate coated with agar containing the test organism Staphylococcus aureus. Samples of the glove material incorporating triclosan were cut and placed on the surface of the agar. The samples were 1.5cm squares. After 30 minutes at room temperature the plates were incubated at 37°C for 24 to 48 hours and the zone of inhibition of growth round the samples measured. An enhanced zone of inhibition as compared to a sample of non-triclosan containing glove material showed effective release of triclosan.

### Example 2

### Preparation of a Glove

A dispersion of triclosan in water (at 40% solids) was formed by ball milling for 5 hours the following mixture:

| | |
|---|---|
| Triclosan | 100g |
| DARVAN No.1* | 20g |
| Ammonium caseinate (10% soln) | 20g |
| Water | to 250g |

| | |
|---|---|
| * sodium salts of polymerised alkyl nephthalene sulphonic acid (20% solution) | |

This dispersion was mixed with an aqueous rubber latex (solids content 41.5%) in the following proportions:

| | |
|---|---|
| 40% Triclosan dispersion | 8.3g |
| Latex | to 800g |

The two components were mixed until a homogeneous mixture was achieved.

A conventional aqueous calcium nitrate-calcium carbonate coagulation solution was prepared.

A glove former, preheated to 112°C was dipped into the coagulant solution, removed and air-dried for about 1-2 minutes. The coated former was then immersed in the prevulcanised natural rubber latex containing triclosan. The latex coated former was then withdrawn and air dried from 1½ to 2 minutes. The latex coated former was then immersed for 2 minutes in a tank of water heated to about 70°C to extract any water soluble materials. The glove was cured at 115°C for about 25 minutes. After cooling the glove was dusted with corn starch powder and stripped from the former.

### Example 3

### Preparation of Dusting Powder

A 0.17% w/w solution of chlorhexidine digluconate in water (25g) were mixed with maize starch (23.75g). The resulting mixture was dried in an oven at 70°C for four hours. The resulting solid mass was ground in a pestle and mortar and then sieved through a 150»m sieve so that the dusting powder has a granular size of less than 150»m diameter. The chlorhexidine digluconate solution was prepared by taking a commercially available chlorhexidine gluconate solution (0.927g) containing 18.54% w/w chlorhexidine digluconate and diluting to 100g with water.

### Preparation of a Glove

Triclosan was incorporated into a natural rubber latex employing the process described in Example 1.

A glove was formed by the process described in Example 1.

The glove was cured, dusted with chlorhexidine digluconate-containing maize starch and stripped from the former.

A sample of the glove material was taken and tested for release of the antibacterial agents using the method described herein before. An enhanced zone of inhibition as compared to a sample of non-antibacterial agent containing glove material showed effective release of the antibacterial agents.

### Example 4

### Preparation of Dusting Powder

A 5% w/w solution of triclosan in acetone (10g) were mixed with maize starch (24.5g). The resulting mixture was dried in an oven at 50°C for four hours. The resulting solid mass was ground in a pestle and mortar and then sieved through a 150»m sieve so that the dusting powder has a granular size of less than 150»m diameter. The triclosan solution was prepared by taking triclosan powder (5g) and dissolving in acetone (95g).

### Preparation of Glove

A glove of natural rubber latex incorporating triclosan (1%) was prepared as described in Example 2. A glove on the former had the dusting powder applied to it (on what would be its inside surface) using a small fine haired brush. Sufficient powder was applied over the whole surface to prevent the glove sticking to itself, 0.7g is a suitable amount.

A sample of the glove material to which had been applied the dusting powder was placed in the centre of a plate containing an agar growing medium seeded with Staphylococcus aureus. The plate was incubated and a zone of inhibition of growth of the bacteria around the sample was observed indicating successful release of the antibacterial agent.

### Example 5

### Preparation of Dusting Power

A triclosan-βcyclodextrin complex was prepared as follows :
β-cyclodextrin (18.75g) was placed in a 3-necked flask together with distilled water (100 ml) and heated with stirring until the cyclodextrin had dissolved. Triclosan (4.34g) was dissolved in sodium hydroxide solution (20 ml, 1 molar) and was added to the flask over a period of 30 minutes with stirring. Ethanol (30ml as 3 x 10 ml aliquots) was added and the reaction mixture heated and stirred for a further 2 hours. The reaction mixture was then allowed to cool with stirring to room temperature. The alkaline solution was treated with hydrochloric acid (0.5m) until the pH of the solution was 3.5. A white precipitate was separated from the resulting solution by centrifuging. The precipitate was washed with a small volume of water three times, centrifuging between each wash to remove inorganic materials such as sodium chloride. The precipitate was dried at 65°C in a vacuum oven for 1 hour. The complex comprising the precipitate was then used in the dusting powder. The complex (4.8g) was mixed with maize starch (45.2g) in a ball mill. The resulting powder contained 2% w/w of triclosan.

A glove incorporating triclosan was coated with the dusting powder.

A sample of the coated glove material exhibited antibacterial activity through release of triclosan from the glove and dusting powder.

### Example 6

### Preparation of Dusting Power

A chlorhexidine digluconate-βcyclodextrin complex was prepared as follows :
β-cyclodextrin (11.43g) was placed in a 3-necked flask together with distilled water (75 ml) and heated with stirring until the cyclodextrin had dissolved. Chlorhexidine diglucoate (48.9g of an 14.48% w/w aqueous solution) was added to the flask over a period of 30 minutes with stirring. After the addition was complete the solution was heated for a further 15 minutes and allowed to cool with stirring to room temperature. The solution was then placed in a refrigerator at 5°C until precipitation of a white solid was completed. The precipitation was filtered off and dried. The complex comprising the precipitate was then used in the dusting powder. The complex (5.7g) was mixed with maize starch (44.3g) in a ball mill. The resulting powder contained 5% w/w of chlorhexidine digluconate.

A glove was coated with the dusting powder.

A sample of the glove material to which had been applied the dusting powder was placed in the centre of a plate containing an agar growing medium seeded with Staphylococcus aureus. The plate was incubated and a zone of inhibition of growth of the bacteria around the sample was observed indicating successful release of the antibacterial agent.

### Example 7

### Preparation of Dusting Powder

A solution of chlorhexidine digluconate is freeze dried to provide solid particles of chlorhexidine digluconate. These particles are mixed with dry maize or calcium carbonate powder in a ball mill to provide a dry powder containing chlorhexidine digluconate.

The dry powder may be applied to the inside surface of a glove incorporating triclosan (1%) prepared by the method described in Example 2

## Claims

1. A method for the manufacture of antimicrobial releasing thin natural rubber latex gloves, including the step of incorporating an antimicrobially effective amount of a non-ionic, sparingly water soluble antimicrobial agent into the glove material prior to forming the glove.

2. A method as claimed in claim 1 wherein the antimicrobial agent is 2,4,4' - trichloro - 2-hydroxydiphenyl ether.

3. A method as claimed in claim 1 or claim 2 wherein the glove material contains from 0.1 to 10% by weight of antimicrobial agent.

4. A method for the manufacture of antimicrobial releasing thin polymer gloves which includes the step of dipping a glove former into a first polymer solution or latex containing from 0.1 to 10% by weight of a non-ionic sparingly water soluble antimicrobial agent.

5. A method as claimed in claim 4 wherein the glove former is precoated with a polymer.

6. A method as claimed in claim 4 or claim 5 which includes the further step of dipping a former, coated with said antimicrobial agent containing polymer, into a second polymer solution or latex.

7. An antimicrobial releasing thin natural rubber latex glove containing an antimicrobially effective non-ionic, sparingly water soluble amount of antimicrobial agent.

8. An antimicrobial releasing thin natural rubber latex glove containing an antimicrobially effective amount of 2,4,4' - trichloro - 2' hydroxydiphenyl ether.

9. A glove as claimed in claim 7 or 8 which has on at least the wearer-contacting surface thereof a powder containing an antimicrobially effective amount of an antimicrobial agent.

10. A glove as claimed in claim 9 in which the antimicrobial agent is chlorhexidine digluconate.

11. A glove as claimed in claim 9 in which the antimicrobial agent is triclosan.

12. A glove as claimed in claim 10 or claim 11 in which the powder also comprises a starch.

13. An antimicrobial releasing thin natural rubber latex glove containing an antimicrobially effective amount of 2,4,4' - trichloro - 2 - hydroxydiphenyl ether and on at least the inside or wearer-contacting surface thereof, a powder comprising an antimicrobially effective amount of a complex of chlorhexidine digluconate and cyclodextrin.

## Patentansprüche

1. Verfahren zum Herstellen von ein bakterienhemmendes Mittel freisetzenden Handschuhen aus dünnem Natur-Kautschuklatex, welches den Schritt einschließt, eine bakterienhemmend wirksame Menge eines nichtionischen, in Wasser schwerlöslichen bakterienhemmenden Wirkstoffes dem Handschuhmaterial vor der Formung des Handschuhs beizumengen.

2. Verfahren gemäß Anspruch 1, dadurch gekennzeichnet, daß der bakterienhemmende Wirkstoff 2,4,4'-Trichlor-2-Hydroxydiphenyl-Ether ist.

3. Verfahren gemäß Anspruch 1 oder 2, dadurch gekennzeichnet, daß das Handschuhmaterial 0,1 bis 10 Gew.-% des bakterienhemmenden Wirkstoffes enthält.

4. Verfahren zum Herstellen von ein bakterienhemmendes Mittel freisetzenden Handschuhen aus dünnem Polymer, welches den Schritt einschließt, eine Handschuhform in eine erste Polymerlösung oder Latex zu tauchen, die bzw. das 0,1 bis 10 Gew.-% eines nichtionischen, in Wasser schwerlöslichen bakterienhemmenden Wirkstoffes enthält.

5. Verfahren nach Anspruch 4, dadurch gekennzeichnet, daß die Handschuhform mit einem Polymer vorbeschichtet wird.

6. Verfahren nach Anspruch 4 oder 5, welches einen weiteren Schritt einschließt, in welchem eine Handschuhform, die mit dem einen bakterienhemmenden Wirkstoff enthaltenden Polymer beschichtet ist, in eine zweite Polymerlösung oder Latex getaucht wird.

7. Ein bakterienhemmendes Mittel freisetzender Handschuh aus dünnem Natur-Kautschuklatex, welcher eine bakterienhemmend wirksame Menge eines nichtionischen, in Wasser schwerlöslichen bakterienhemmenden Wirkstoffes enthält.

8. Ein bakterienhemmendes Mittel freisetzender Handschuh aus dünnem Natur-Kautschuklatex, welcher eine bakterienhemmend wirksame Menge an 2,4,4'-Trichlor-2'-Hydroxydiphenyl-Ether enthält.

9. Handschuh gemäß Anspruch 7 oder 8, welcher wenigstens auf der mit dem Benutzer in Kontakt kommenden Oberfläche mit einem Puder versehen ist, der eine bakterienhemmend wirksame Menge eines bakterienhemmenden Wirkstoffes enthält.

10. Handschuh gemäß Anspruch 9, dessen bakterienhemmender Wirkstoff Chlorhexidin-Digluconat ist.

11. Handschuh gemäß Anspruch 9, dessen bakterienhemmender Wirkstoff Triclosan ist.

12. Handschuh gemäß Anspruch 10 oder 11, bei welchem der Puder auch eine Stärke enthält.

13. Ein bakterienhemmendes Mittel freisetzender Handschuh aus dünnem Natur-Kautschuklatex, welcher eine bakterienhemmend wirksame Menge an 2,4,4'-Trichlor-2-Hydroxydiphenyl-Ether enthält und wenigstens auf der Innenseite oder der mit dem Benutzer in Kontakt kommenden Oberfläche einen Puder aufweist, welcher eine bakterienhemmend wirksame Menge eines Komplexes aus Chlorhexidin-Digluconat und Cyclodextrin enthält.

## Revendications

1. Procédé pour la fabrication de gants minces en latex de caoutchouc naturel libérant un agent antimicrobien, comprenant l'étape d'incorporation d'une quantité efficace du point de vue antimicrobien d'un agent antimicrobien non ionique, faiblement soluble dans l'eau, dans le matériau du gant avant formage du gant.

2. Procédé suivant la revendication 1, dans lequel l'agent antimicrobien est l'éther 2,4,4'-trichloro-2-hydroxydiphénylique.

3. Procédé suivant les revendications 1 ou 2, dans lequel le matériau du gant contient de 0,1 à 10% en poids d'agent antimicrobien.

4. Procédé pour la fabrication de gants minces en polymère libérant un agent antimicrobien, comprenant l'étape d'immersion d'un formeur de gants dans une première solution de polymère ou un latex contenant de 0,1 à 10% en poids d'un agent antimicrobien non ionique, faiblement soluble dans l'eau.

5. Procédé suivant la revendication 4, dans lequel le formeur de gants est pré-enduit avec un polymère.

6. Procédé suivant les revendications 4 ou 5, qui comprend l'étape supplémentaire d'immersion d'un formeur revêtu d'un polymère contenant cet agent antimicrobien dans une seconde solution de polymère ou un latex.

7. Gant mince en latex de caoutchouc naturel libérant un agent anti-microbien contenant une quantité efficace du point de vue antimicrobien d'un agent antimicrobien non ionique, faiblement soluble dans l'eau.

8. Gant mince en latex de caoutchouc naturel libérant un agent antimicrobien contenant une quantité efficace du point de vue antimicrobien d'éther 2,4,4'-trichloro-2-hydroxydiphénylique.

9. Gant suivant les revendications 7 ou 8, dont une surface au moins en contact avec l'utilisateur porte une poudre contenant une quantité efficace du point de vue antimicrobien d'un agent antimicrobien.

10. Gant suivant la revendication 9, dans lequel l'agent antimicrobien est le digluconate de chlorhexidine.

11. Gant suivant la revendication 9, dans lequel l'agent antimicrobien est le triclosan.

12. Gant suivant les revendications 10 ou 11, dans lequel la poudre comprend aussi un amidon.

13. Gant mince en latex de caoutchouc naturel libérant un agent antimicrobien contenant une quantité efficace du point de vue antimicrobien d'éther 2,4,4'-trichloro-2-hydroxydiphénylique et, dont au moins l'intérieur ou la surface en contact avec l'utilisateur porte une poudre contenant une quantité efficace du point de vue antimicrobien d'un complexe de digluconate de chlorhexidine et de cyclodextrine.
